# EUROPÄISCHE PATENTANMELDUNG

(11) **EP 1 437 117 A1**
(43) Veröffentlichungstag der Anmeldung: **14.07.2004**
(21) Anmeldenummer: 02293157.0
(22) Anmeldetag: 19.12.2002
(51) Int. Cl.: A61K 7/00

(54) **Verwendung von Sinapinsäure und/oder Sinapinsäurederivaten**

(71) Anmelder: Cognis France S.A., 31360 Saint-Martory (FR)
(72) Erfinder: Moussou, Philippe, 54000 Nancy (FR); Danoux, Louis, 54420 Saulxures-Les-Nancy (FR); Jeanmaire, Christine, 54000 Nancy (FR); Pauly, Gilles, 54000 Nancy (FR)
(74) Vertreter: Livet, Marie-José

(57) **Zusammenfassung**

Verwendung von Sinapinsäure und/oder Sinapinsäurederivaten zur Herstellung kosmetischer und/oder dermopharmazeutischer Zubereitungen zum Schutz gegen Hautalterung und Faltenbildung, insbesondere durch UV-Strahlung, zur Herstellung kosmetischer und/oder dermopharmazeutischer Zubereitungen zur Verzögerung der Alterung von Mitochondrien, zum Schutz des mitochondrialen Genoms vor oxidativem Stress, zur Verbesserung der Energiegewinnungskapazität der menschlichen Haut, die Verwendung von Sinapinsäure und/oder Sinapinsäurederivaten zur Herstellung kosmetischer und/oder dermopharmazeutischer Zubereitungen zum Schutz der zellulären DNA, zum Schutz vor Thymin-Dimerbildung nach Einfluß von UV-B-Strahlung in Hautzellen und zur Vesserung des DNA-Reparaturmechanismus in Hautzellen, zur Stimulation der Collagensynthese, sowie die Verwendung zur Herstellung von Zubereitungen zur Wundheilung nach nicht enzymatischer Glycosylierung oder Einfluß von oxidativem Stress oder Umweltgiften.

## Beschreibung

### Gebiet der Erfindung

Die vorliegende Erfindung bezieht sich auf das Gebiet der Kosmetik und der Dermopharmazie. Sie beschreibt die Verwendung von Sinapinsäure und/oder Sinapinsäurederivaten in kosmetischen und/oder pharmazeutischen Zubereitungen zum Schutz der Haut gegen Hautalterung, besonders durch UV-Strahlung und/oder zum Schutz gegen Faltenbildung. Insbesondere wird die Verwendung von Sinapinsäure und/oder Sinapinsäurederivaten in kosmetischen und/oder pharmazeutischen Zubereitungen zum Schutz von dermalen Mitochondrien, zellulärer DNA und dem dermalen Immunsystem.

### Stand der Technik

Sinapinsäure ist ein Derivat der Zimtsäure und als Antioxidans besonders zur Vermeidung der Oxidation von Lipiden in Lebensmitteln bekannt. Das UV-Absorptionsmaximum der Verbindung in Ethanol liegt bei 323 nm und ist damit vergleichbar den Benzophenonen, die typische UV-Filter in Kosmetika darstellen. Jedoch beobachtet man bei Einsatz von Sinapinsäure im Gegensatz zu herkömmlichen Benzophenonen eine geringere Bildung von Erythemen. Auch die Inhibierung der Tyrosinaseaktivität konnte für Sinapinsäure und ihre Derivate nachgewiesen werden. Daher werden in der japanischen Patentanmeldung JP 01013017 Sinapinsäure und ihre Derivate als Hautweißungsmittel und UV-Filter offenbart.

Bereits 1993 konnte eine anti-inflammatorische Wirkung im Carrageenan-induzierten Rattenpfotenödem-Modell nach Injektion in die Pfote für Sinapinsäure und ihre Ester beobachtet werden [Chawla AS et al, Indian J Pharma Sci (1993) 55, p 184-7]. Weitere Anwendungen dieser Substanz als Cholinacetyltransferase-Aktivator zur Verbesserung der Cerebralfunktion [JP 07179338] und als antimikrobielles Konservierungsmittel in Lebensmitteln [JP 95194356] zeigen die vielfältigen Einsatzmöglichkeiten von Sinapinsäure und ihren Derivaten.

Die Verwendungen verwandter Derivate der Zimtsäure zur Stärkung der Barrierefunktion und Hydratation der Haut, sowie zur Stimulation der Lipid- und der Collagensynthese sind aus der Europäischen Patentanmeldung EP 0 925 779 bekannt. Diese Derivate können entsprechend der EP 0 962 223 auch zur beschleunigten Erneuerung der Epidermis und zur Behandlung gegen die Hautalterung eingesetzt werden.

Antioxidative Substanzen haben sich auch in der Kosmetik bewährt, wobei sie nicht nur zum Schutz von Wirkstoffen in Zubereitungen eingesetzt werden, sondern selber wirksame Aktivsubstanzen darstellen können. Es besteht im Markt ein ständiges Bedürfnis nach Produkten mit einem verbesserten Leistungsspektrum. Hierbei sind Hautverträglichkeit sowie der Einsatz natürlicher Produkte beim Kunden gefragt. Daneben ist es wünschenswert durch das Auffinden neuer Einsatzgebiete bereits bekannter Substanzklassen deutlich bessere Produkte zu erhalten. Besonders Extrakte von nachwachsenden Rohstoffen und deren Inhaltstoffe finden immer häufiger Einsatz in der Kosmetik.

Die komplexe Aufgabe der vorliegenden Patentanmeldung hat daher darin bestanden, diesen Anforderungen Rechnung zu tragen und auf dem Gebiet bereits bekannter Inhaltsstoffe neue Wirkungen für bisher unbekannte Einsatzgebiete auszunutzen.

### Beschreibung der Erfindung

Gegenstand der vorliegenden Erfindung ist die Verwendung von Sinapinsäure und/oder Sinapinsäurederivaten zur Herstellung kosmetischer und/oder dermopharmazeutischer Zubereitungen zum Schutz gegen Hautalterung, insbesondere zum Schutz gegen Hautalterung durch UV-Strahlung und/oder zum Schutz gegen Faltenbildung.

Ein weiterer Gegenstand der Erfindung ist die Verwendung von Sinapinsäure und/oder Sinapinsäurederivaten zur Herstellung kosmetischer und/oder dermopharmazeutischer Zubereitungen zur Verbesserung der Wundheilung, insbesondere nach nicht enzymatischer Glycosylierung oder Einfluß von oxidativem Stress oder Umweltgiften.

Das mitochondriale Atmungssystem ist die hauptsächliche intrazelluläre Quelle der reaktiven Sauerstoffspecies (reactive oxygen species - ROS). Während des normalen Alterungsprozesses erfahren die Mitochondrien in den Zellen eine Erhöhung von oxidativem Stress und damit verbundenen oxidativen Zellschädigungen.

Die mitochondriale DNA (Desoxyribonukleinsäure- mtDNA) ist, sobald sie nicht über Histone und DNA-bindende Proteine geschützt wird, kontinuierlich einem hohen Gehalt an ROS und freien Radikalen ausgesetzt, die sich in der Matrix der Mitochondrien ansammeln. Daher unterliegt die mtDNA in hohem Maße oxidativen Modifikationen und Mutationen, die mit zunehmender Alterung exponentiell ansteigen. Als Folge davon enthalten Atmungsenzyme defekte mtDNA kodierte Proteinuntereinheiten und weisen dadurch eine verschlechterte Elektronentransportfunktion und eine erhöhte ROS Produktion auf, die wiederum zu vermehrtem oxidativem Stress und oxidativen Schädigungen von Mitochondrien führt. Dieser Circulus viciosus zeigt die Problematik des wachsenden Verfalls unterschiedlicher Gewebe und Organe während des biologischen Alterungsprozesses.

Mitochondrien in menschlichen Zellen enthalten ungefähr 10 Kopien der schmalen 16,5 kb kreisförmigen Moleküle doppelsträngiger DNA. Die Zerstörung von mitochondrialer DNA ist intensiver und nachhaltiger als die der zellulären DNA in Zellen, die durch oxidativen Stress betroffen sind. Schädigungen mitochondrialer DNA in menschlichen dermalen Fibroblasten durch UV-A-Strahlung oberhalb von 340 nm induzieren eine Akkumulation von verkürzten mtDNA Fragmenten, denen das 4977-bp-Fragment fehlt. Dieser - auch als "common deletion" bezeichnete- Defekt resultiert in einem verminderten mitochondrialen Mem- branpotential, einer geringeren ATP-Synthese und einer ungünstigeren ATP/ADP Rate.

Der Verlust des 4977-bp Fragmentes beeinflusst Gene die 7 Polypeptidkomponenten der mitochondrialen Atmungskette kodieren, sowie 5 der 22 tRNAs, die für die mitochondriale Proteinsynthese benötigt werden. Der Anteil der mtDNA mit fehlenden 4977bp Fragmenten in menschlichen Hautzellen steigt mit zunehmendem Alter und wiederholter UV-A-Exposition und ist beim sogenannten Photo-ageing weit mehr ausgeprägt als beim normalen chronologischen Alterungsprozess [Berneburg M et al, J Invest Dermatol, 1998, 111, pp 709-710; Berneburg M & Krutmann J, Methods in Enzymol, 2000, 319, pp 366-376]

Überraschender Weise wurde nun festgestellt, dass Sinapinsäure und ihre Derivate ein schützende Wirkung auf Adenosindinukleotide (ADN) der Mitochondrien haben. Sie vermindern die Schädigung des mitochondrialen Genoms, die durch oxidativen Stress bewirkt wird und führen dadurch zu einer Verzögerung der Alterung von Mitochondrien und zu einer Verbesserung des Energiepotentials der Zellen. Somit können Sinapinsäure und ihre Derivate über die Verbesserung der mitochondrialen Funktion und Energiebilanz zum Schutz der Haut gegen Alterung und Faltenbildung, zur Steigerung effektiver Reparaturmechanismen durch Alterung und Sonneneinstrahlung geschädigter Haut und zur verbesserten Wundheilung der Haut entscheidend beitragen.

Weitere Gegenstände der Erfindung sind daher auch die Verwendung von Sinapinsäure und/oder Sinapinsäurederivaten zur Herstellung von kosmetischen und/oder dermopharmazeutischen Zubereitungen zum Schutz der cellulären DNA und/oder der Adenosindinukleotide (ADN), zur Verzögerung der Alterung von Mitochondrien, zum Schutz des mitochondrialen Genoms vor oxidativem Stress und zur Verbesserung der Energiegewinnungskapazität der menschlichen Haut.

Setzt man lebende Zellen dem Einfluß von UV-Strahlung aus , so werden Schädigungen der zellulären DNA induziert. Eines der hauptsächlichen durch UV-B-Strahlung induzierte Reaktionsprodukt in den Zellen sind Cyclobutan Pyrimidin Dimere (Thymin-Dimere). Die Exposition mmit UV-Strahlung kann auf diese Weise zu Hautkrebs, Photo-aging und Photodermatosen führen, wobei die Bildung von Thymin-Dimeren der Anfangsschritt einer Kaskade von schädigenden Reaktionen ist, die durch Auslösung von Mutationen oder immunsuppresiven Wirkungen zu weiteren stark Gesundheitsschädigenden Prozessen beiträgt. Hautzellen sind dabei ganz besonders dem Einfluß der UV-Strahlung unterworfen und der Schutz vor Sonnen einstrahlung der Haut ist gleichbedeutend mit einem Schutz der dermalen DNA.

Es konnte festgestellt werden, dass durch die topische Anwendung von Sinapinsäure und ihren Derivaten insbesondere die Bildung von Thymin-Dimeren in zellulärer DNA, die durch UV-Strahlung induziert wird, vermindert wird. Dadurch kann die Anzahl der Mutationen gesenkt und eine Stärkung des dermalen Immunsystems bewirkt werden. Ein weiterer Gegenstand der Erfindung ist daher die Verwendung von Sinapinsäure und/oder Sinapinsäurederivaten zur Herstellung von kosmetischen und/oder dermopharmazeutischen Zubereitungen zum Schutz vor Thymin-Dimerbildung nach Einfluß von UV-Strahlung in Hautzellen und zur Verbesserung des DNA-Reparaturmechanismus in Hautzellen.

Die menschliche Haut enthält vorwiegend zwei Typen von Collagen: Collagen Typ I und Collagen Typ III, synthetisiert durch dermale Fibroblasten. Beide Collagentypen stellen wesentliche Strukturkomponenten der extrazellulären Matrix des Bindegewebes dar und tragen in hohem Maße zur Struktur der gesamten Haut bei. Ein wichtiges Kennzeichen alternder Haut ist die Abnahme des Collagengehaltes in der Dermis und eine damit verbundene Abnahme der Elastizität, die zusätzlich durch eine Verschlechterung der Fibroblastenfunktion durch nicht enzymatische Glycosylierung (Glycation) und oxidativen Abbau bewirkt wird. Es wurde nun festgestellt, dass Sinapinsäure und ihre Derivate die Collagensynthese dermaler Fibroblasten stimulieren und auf diese Weise die chronologische und die UV-Strahlungsinduzierte Hautalterung verzögert werden kann. Die Verwendung von Sinapinsäure und/oder Sinapinsäurederivaten zur Herstellung kosmetischer und/oder dermopharmazeutischer Zubereitungen zur Stimulation der Collagensynthese in dermalen Fibroblasten ist daher ein weiterer Gegenstand der Erfindung.

Die Haut ist ein Gewebe, das mit einer Vielzahl von Nervenfasern durchzogen ist, deren Zellkörper in den dorsalen Ganglien (dorsal root ganglia - DRG) zusammenführen. Neurone des Typs Aδ und C erreichen mit ihren Zellen die Dermis und auch die Epidermis. Typ C Neurone sind für die Detektion von noziceptiven Stimuli verantwortlich. Keratinocyten und Nervenfasern können eine Vielzahl unterschiedlicher Cytokine produzieren, die unter dem Einfluß externer Stimuli ausgeschieden werden und die sensorischen Enden der Nervenfasern stimulieren. Diese setzen dann wiederum Peptide frei, die die metabolische Aktivität der Keratinocyten über Messenger-Moleküle beeinflussen. Auf diese Weise kommunizieren sensorische Nerven und Keratinocyten kontinuierlich miteinander. Eine Disregulation dieses physiologischen Gleichgewichts erklärt viele pathologische Erscheinungen von empfindlicher Haut oder beispielsweise Psoriasis. Es konnte nun festgestellt werden, dass Sinapinsäure und ihre Derivate insbesondere empfindliche Haut dadurch beruhigen, dass sie die Freisetzung von Neuropeptiden wie beispielsweise Substanz P oder CGRP herabsetzen.

Ein weiterer Gegenstand der Erfindung ist daher die Verwendung von Sinapinsäure und/oder Sinapinsäurederivaten zur Herstellung kosmetischer und/oder dermopharmazeutischer Zubereitungen für empfindliche Haut und zur Linderung neurogener Entzündungen

Gegenstände der Erfindung sind ebenfalls die entsprechenden Verfahren zur kosmetischen Behandlung zum Schutz der Haut gegen Alterung und/oder Faltenbildung, zum Schutz von Mitochondrien der Hautzellen gegen Schädigungen durch Alterung, UV-Strahlungseinwirkung, oxidativen Stress und Umweltgifte und ein Verfahren zur Verbesserung der Energiegewinnung von Hautzellen, dadurch gekennzeichnet, dass eine Zubereitung enthaltend Sinapinsäure und/oder Sinapinsäurederivate topisch appliziert wird. Unter topischer Applikation ist im Sinne der vorliegenden Erfindung die lokale Anwendung einer Zubereitung enthaltend Sinapinsäure und/oder Sinapinsäurederivate auf Haut, Kopfhaut und Schleimhaut zu verstehen.

### Sinapinsäure

Sinapinsäure ist ein Derivat der Zimtsäure - 4-Hydroxy-3,5-Dimethoxy-Zimtsäure gemäß der Formel (I).

Synonyme Bezeichnungen sind 2-Propenoic acid, Sinapinic acid und Sinapic acid

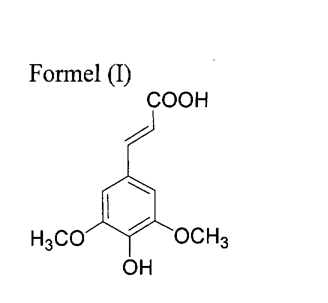

Die Herstellung der Sinapinsäure erfolgt nach herkömmlichen dem Fachmann bekannten Methoden.

Die erfindungsgemäßen Zubereitungen enthalten Sinapinsäure und/oder Sinapinsäurederivate in Mengen von 0,0001 bis 10 Gew.%, vorzugsweise in Mengen von 0,001 bis 5 Gew. % und besonders bevorzugt in Mengen von 0,01 bis 3 Gew.% bezogen auf die Gesamtzusammensetzung der Zubereitung.

Als Derivate der Sinapinsäure kommen Sinapinsäuresalze oder Derivate als Ester, Aldehyde oder Alkohole mit linearen, verzweigten cyclischen, polycyclischen Alkyl-, Alkenyl-, Aryl-, Hydroxy-, Amin-, Thiol-, quartären Aminogruppen oder Verbindungen der Sinapinsäure mit Polyolen, Zuckern, Aminozuckern, Aminosäuren oder quartären Aminen in Betracht.

Vorzugsweise wird jedoch die reine, nicht derivatisierte Sinapinsäure und deren Salze eingesetzt. Leider ist die chemische Stabilität der reinen Sinapinsäure in kosmetischen Zubereitungen kritisch zu beurteilen, da sich Sinapinsäure unter dem Einfluß von Licht, Sauerstoff, Oxidationsmitteln, Metallionen, Temperaturerhöhung oder Wasser leicht zersetzt. Die entstehenden Abbauprodukte bilden gefärbte übelriechende Substanzen, die auch zu Hautreizungen führen können. Es ist daher ein weiterer Gegenstand der Erfindung Sinapinsäure und/oder ihre Derivate in verkapselter, komplexierter oder adsorbierter Form in den Zubereitungen einzusetzen. Dabei bietet sich insbesondere die Verkapselung in Liposomen oder einer hydrophoben Matrix, die Komplexierung in Cyclodextrinen und die Adsorption an hydrophobe Träger an. Bevorzugt wird Sinapinsäure und/oder ihre Derivate in Mikrokapseln eingearbeitet.

Zusätzlich können den Zubereitungen weitere Substanzen wie UV-Filter, andere Antioxidantien oder Metallkomplexbildner zugesetzt werden, um Schwermetalle und freie Radikale an einem Abbau der Sinapinsäure und ihrer Derivate zu hindern.

### Mikrokapseln

Zum Schutz der Aktivsubstanzen gegen Licht, Oxidation und Temperaturerhöhung hat es sich als nützlich erwiesen, die Zubereitungen zu verkapseln. Die Freisetzung des Wirkstoffes erfolgt bei direkter Anwendung auf der Haut durch mechanische Einwirkung auf die Membran beim Auftragen.

Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlos-sen ist. Genauer gesagt handelt es sich um mit filmbildenden Polymeren umhüllte feindisper-se flüssige oder feste Phasen, bei deren Herstellung sich die Polymere nach Emulgierung und Koazervation oder Grenzflächenpolymerisation auf dem einzuhüllenden Material niederschla-gen. Nach einem anderen Verfahren werden geschmolzene Wachse in einer Matrix aufge-nommen ("microsponge"), die als Mikropartikel zusätzlich mit filmbildenden Polymeren um-hüllt sein können. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natriumoder Calciumal-ginat, Fette und Fettsäuren, Cetylalkohol, Collagen, Chitosan, Lecithine, Gelatine, Albumin, Schellack, Polysaccaride, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind bei-spielsweise Polymere wie Polyacrylate, Polyamide, Polyvinylalkohol oder Polyvinylpyrrolidon.

### Gewerbliche Anwendung

Die erfindungsgemäßen kosmetischen und/oder pharmazeutischen Zubereitungen enthalten Sinapinsäure und/oder Sinapinsäurederivate in Mengen von 0,0001 bis 10 Gew.%, vorzugsweise 0,001 bis 5 Gew.% und besonders bevorzugt 0,01 bis 3 Gew.% .

Die durch Formel (I) beschriebene Sinapinsäure und ihre Derivate können zur Herstellung kosmetischer und/oder pharmazeutischer Mittel, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben, vornehmlich jedoch Erzeugnisse für Gesicht und Körper, Tag- oder Nachtkosmetika, Sonnenschutzmittel, kräftigende, regenerierende Erzeugnisse, Anti-Faltenkosmetika, Schlankheitskurenmittel und Mittel gegen Alterungsprozesse dienen.

Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, weitere Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, a-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, a-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen oder verzweigten C6-C22-Fettalkoholen bzw. Ester von verzweigten C6-C13-Carbonsäuren mit linearen oder verzweigten C6-C22-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C6-C22-Fettsäuren mit verzweigten Alkoholen, insbesondere 2-Ethylhexanol, Ester von C18-C38-Alkylhydroxycarbonsäuren mit linearen oder verzweigten C6-C22-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, Ester von C2-C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C6-C22-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C6-C22-Alkoholen (z.B. Finsolvâ TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Ø: Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Ø: Alkyl- und/oder Alkenyloligoglykoside mit 8 bis 22 Kohlenstoffatomen im Alk(en)ylrest und deren ethoxylierte Analoga;
- Ø: Anlagerungsprodukte von 1 bis 15 Mol Ethylenoxid an Ricinusöl und/oder gehärtetes Ricinusöl;
- Ø: Anlagerungsprodukte von 15 bis 60 Mol Ethylenoxid an Ricinusöl und/oder gehärtetes Ricinusöl;
- Ø: Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Ø: Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Ø: Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Ø: Mono-, Di- und Trialkylphosphate sowie Mono-, Di- und/oder Tri-PEGalkylphosphate und deren Salze;
- Ø: Wollwachsalkohole;
- Ø: Polysiloxan-Polyalkyl-Polyether-Copolymere bzw. entsprechende Derivate;
- Ø: Block-Copolymere z.B. Polyethylenglycol-30 Dipolyhydroxystearate;
- Ø: Polymeremulgatoren, z.B. Pemulen-Typen (TR-1,TR-2) von Goodrich;
- Ø: Polyalkylenglycole sowie
- Ø: Glycerincarbonat.

### Ø Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C12/18-Fettsäuremono- und -diester von Anlagerungsprodukten von Ethylenoxid an Glycerin sind als Rückfettungsmittel für kosmetische Zubereitungen bekannt.

### Ø Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### Ø Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### Ø Sorbitanester

Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### Ø Polyglycerinester

Typische Beispiele für geeignete Polyglycerinester sind Polyglyceryl-2 Dipolyhydroxystearate (Dehymulsâ PGPH), Polyglycerin-3-Diisostearate (Lameformâ TGI), Polyglyceryl-4 Isostearate (Isolanâ GI 34), Polyglyceryl-3 Oleate, Diisostearoyl Polyglyceryl-3 Diisostearate (Isolanâ PDI), Polyglyceryl-3 Methylglucose Distearate (Tego Careä 450), Polyglyceryl-3 Beeswax (Cera Bellinaâ), Polyglyceryl-4 Caprate (Polyglycerol Caprate T2010/90), Polyglyceryl-3 Cetyl Ether (Chimexaneâ NL), Polyglyceryl-3 Distearate (Cremophorâ GS 32) und Polyglyceryl Polyricinoleate (Admulâ WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Diund Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### Ø Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### Ø Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonium-glycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C8/18-Alkyl- oder Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C12/18-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-snglycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopoleâ und Pemulen-Typen von Goodrich; Synthaleneä von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400ä von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquatâ (BASF), Kondensationsprodukte von Polyglycolen und Aminen, quaternierte Kollagenpolypeptide, wie beispielsweise Lauryldimonium Hydroxypropyl Hydrolyzed Collagen (LamequatâL/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretineâ/Sandoz), Copolymere der Acrylsäure mit Dimethyl-diallylammoniumchlorid (Merquatâ 550/Chemviron), Polyaminopolyamide, sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguarâ CBS, Jaguarâ C-17, Jaguarä C-16 der Firma Celanese, quaternierte Ammoniumsalz-Polymere, wie z.B. Mirapolâ A-15, Mirapolä AD-1, Mirapolä AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und weitere Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- Ø: 3-Benzylidencampher bzw. 3-Benzylidennorcampher und dessen Derivate, z.B. 3-(4-Methylbenzyliden)campher;
- Ø: 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
- Ø: Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ø: Ester der Salicylsäure, vorzugsweise Salicylsäure-2-ethylhexylester, Salicylsäure-4-iso-propylbenzylester, Salicylsäurehomomenthylester;
- Ø: Derivate des Benzophenons, vorzugsweise 2-Hydroxy-4-methoxybenzophenon, 2-Hydroxy-4-methoxy-4' -methylbenzophenon, 2,2' -Dihydroxy-4-methoxybenzophenon;
- Ø: Ester der Benzalmalonsäure, vorzugsweise 4-Methoxybenzmalonsäuredi-2-ethylhexyl-ester;
- Ø: Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorbâ HEB);
- Ø: Propan-1,3-dione, wie z.B. 1-(4-tert.Butylphenyl)-3-(4'methoxyphenyl)propan-1,3-dion;
- Ø: Ketotricyclo(5.2.1.0)decan-Derivate.

Als wasserlösliche Substanzen kommen in Frage:
- Ø: 2-Phenylbenzimidazol-5-sulfonsäure und deren Alkali-, Erdalkali-, Ammonium-, Alkylammonium-, Alkanolammonium- und Glucammoniumsalze;
- Ø: Sulfonsäurederivate von Benzophenonen, vorzugsweise 2-Hydroxy-4-methoxybenzo-phenon-5-sulfonsäure und ihre Salze;
- Ø: Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsolä 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'methoxydibenzoylmethan (Parsolâ 1789) und 2-Cyano-3,3-phenylzimtsäure-2-ethylhexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. a-Carotin, b-Carotin, Lycopin) und deren Derivate, Chlorogensäure und deren Derivate, Liponsäure und deren Derivate (z.B.

Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, g-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mmol/kg), ferner (Metall)-Chelatoren (z.B. a-Hydroxyfettsäuren, Palmitinsäure, Phytinsäure, Lactoferrin), a-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. g-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, a-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### Ø Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### Ø Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagenâ CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### Ø Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, b-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### Ø Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- Ø: adstringierende Wirkstoffe,
- Ø: Ölkomponenten,
- Ø: nichtionische Emulgatoren,
- Ø: Coemulgatoren,
- Ø: Konsistenzgeber,
- Ø: Hilfsstoffe wie z. B. Verdicker oder Komplexierungsmittel und/oder
- Ø: nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkoniumpentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- Ø: entzündungshemmende, hautschützende oder wohlriechende ätherische Öle,
- Ø: synthetische hautschützende Wirkstoffe und/oder
- Ø: öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), KetoconazolÒ, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lameponâ UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Ø: Glycerin;
- Ø: Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- Ø: technische Oligoglyceringemische mit einem Eigenkondensationsgrad von 1,5 bis 10 wie etwa technische Diglyceringemische mit einem Diglyceringehalt von 40 bis 50 Gew.-%;
- Ø: Methyolverbindungen, wie insbesondere Trimethylolethan, Trimethylolpropan, Trimethylolbutan, Pentaerythrit und Dipentaerythrit;
- Ø: Niedrigalkylglucoside, insbesondere solche mit 1 bis 8 Kohlenstoffen im Alkylrest, wie beispielsweise Methyl- und Butylglucosid;
- Ø: Zuckeralkohole mit 5 bis 12 Kohlenstoffatomen, wie beispielsweise Sorbit oder Mannit,
- Ø: Zucker mit 5 bis 12 Kohlenstoffatomen, wie beispielsweise Glucose oder Saccharose;
- Ø: Aminozucker, wie beispielsweise Glucamin;
- Ø: Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, a-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, b-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

## Patentansprüche

1. Verwendung von Sinapinsäure und/oder Sinapinsäurederivaten zur Herstellung kosmetischer und/oder dermopharmazeutischer Zubereitungen zum Schutz gegen Hautalterung.

2. Verwendung von Sinapinsäure und/oder Sinapinsäurederivaten zur Herstellung kosmetischer und/oder dermopharmazeutischer Zubereitungen zum Schutz gegen Faltenbildung.

3. Verwendung von Sinapinsäure und/oder Sinapinsäurederivaten zur Herstellung kosmetischer und/oder dermopharmazeutischer Zubereitungen zum Schutz gegen Hautalterung durch UV-Strahlung.

4. Verwendung von Sinapinsäure und/oder Sinapinsäurederivaten zur Herstellung kosmetischer und/oder dermopharmazeutischer Zubereitungen zur Verbesserung der Wundheilung, insbesondere nach nicht enzymatischer Glycosylierung oder Einfluß von oxidativem Stress oder Umweltgiften.

5. Verwendung von Sinapinsäure und/oder Sinapinsäurederivaten zur Herstellung kosmetischer und/oder dermopharmazeutischer Zubereitungen zur Verzögerung der Alterung von Mitochondrien.

6. Verwendung von Sinapinsäure und/oder Sinapinsäurederivaten zur Herstellung kosmetischer und/oder dermopharmazeutischer Zubereitungen zum Schutz des mitochondrialen Genoms vor oxidativem Stress.

7. Verwendung von Sinapinsäure und/oder Sinapinsäurederivaten zur Herstellung von kosmetischen und dermopharmazeutischen Zubereitungen zur Verbesserung der Energiegewinnungskapazität der menschlichen Haut.

8. Verwendung von Sinapinsäure und/oder Sinapinsäurederivaten zur Herstellung kosmetischer und/oder dermopharmazeutischer Zubereitungen zum Schutz der zellulären DNA und/oder der Adenosindinukleotide (ADN).

9. Verwendung von Sinapinsäure und/oder Sinapinsäurederivaten zur Herstellung kosmetischer und/oder dermopharmazeutischer Zubereitungen zum Schutz vor Thymin-Dimerbildung nach Einfluß von UV-Strahlung in Hautzellen.

10. Verwendung von Sinapinsäure und/oder Sinapinsäurederivaten zur Herstellung kosmetischer und/oder dermopharmazeutischer Zubereitungen zur Verbesserung des DNA-Reparaturmechanismus in Hautzellen.

11. Verwendung von Sinapinsäure und/oder Sinapinsäurederivaten zur Herstellung kosmetischer und/oder dermopharmazeutischer Zubereitungen zur Stimulation der Collagensynthese in dermalen Fibroblasten.

12. Verwendung von Sinapinsäure und/oder Sinapinsäurederivaten zur Herstellung kosmetischer und/oder dermopharmazeutischer Zubereitungen für empfindliche Haut und zur Linderung neurogener Entzündungen.

13. Verwendung nach mindestens einem der Ansprüche 1 bis 12, **dadurch gekennzeichnet, dass** Sinapinsäure und/oder Sinapinsäurederivate in den Zubereitungen in Mengen von 0,0001 bis 10 Gew. % bezogen auf die Gesamtzubereitung enthalten sind.

14. Verwendung nach mindestens einem der Ansprüche 1 bis 13, **dadurch gekennzeichnet, dass** die Sinapinsäure und/oder Sinapinsäurederivate in den Zubereitungen in mikroverkapselter, komplexierter oder an einen Träger adsorbierter Form vorliegen.

15. Verwendung nach mindestens einem der Ansprüche 1 bis 14, **dadurch gekennzeichnet, dass** als Derivate der Sinapinsäure Sinapinsäuresalze oder Derivate als Ester, Aldehyde oder Alkohole mit linearen, verzweigten cyclischen, polycyclischen Alkyl-, Alkenyl-, Aryl-, Hydroxy-, Amin-, Thiol-, quartären Aminogruppen oder Verbindungen der Sinapinsäure mit Polyolen, Zuckern, Aminozuckern, Aminosäuren oder quartären Aminen eingesetzt werden.

16. Verfahren zur kosmetischen Behandlung zum Schutz der Haut gegen Alterung und/oder Faltenbildung, **dadurch gekennzeichnet, dass** eine Zubereitung enthaltend Sinapinsäure und/oder Sinapinsäurederivate topisch appliziert wird.

17. Verfahren zur kosmetischen Behandlung zum Schutz von Mitochondrien der Hautzellen gegen Schädigungen durch Alterung, UV-Strahlungseinwirkung, oxidativen Stress und Umweltgifte, **dadurch gekennzeichnet, dass** eine Zubereitung enthaltend Sinapinsäure und/oder Sinapinsäurederivate topisch appliziert wird.

18. Verfahren zur Verbesserung der Energiegewinnung von Hautzellen, **dadurch gekennzeichnet, dass** eine kosmetische Zubereitung enthaltend Sinapinsäure und/oder Sinapinsäurederivate topisch appliziert wird.
